# EUROPEAN PATENT APPLICATION

(11) **EP 3 058 865 A1**
(43) Date of publication of application: **24.08.2016**
(21) Application number: 15156186.7
(22) Date of filing: 23.02.2015
(51) Int. Cl.: A61B 5/00, A61F 2/30, A61F 2/48

(54) **In vivo implant diagnostic device**

(71) Applicant: Otto-von-Guericke-University, 39106 Magdeburg (DE)
(72) Inventor: Friebe, Michael, 45657 Recklinghausen (DE); Boese, Axel, 39106 Magdeburg (DE)

(57) **Abstract**

The present invention relates to implant devices and systems for providing *in vivo* and *in vitro* diagnostics of physiological and mechanical parameters of a subject and the implant, respectively.

## Description

### FIELD OF THE INVENTION

The present invention relates to implant devices and systems for providing *in vivo* and *in vitro* diagnostics of physiological and mechanical parameters of a subject and the implant, respectively.

### BACKGROUND ART

Patients are receiving more hip and knee replacement surgeries, with about 600,000 knee replacement procedures alone performed in the US annually. Combined with a growing number of knee replacements in younger people, it is predicted that the amount of hip and knee failures may cost insurers and employers billions of dollars in the coming years, according to The International Herald Tribune (December 2011). Patients with hip and/or knee replacement failure have reported some of the following problems: breakage, mainly metal breaks from constant weight-bearing stress, fractures typically near the artificial joint, loosening of the attachment between the bone and artificial device, both in cemented and un-cemented artificial joints, painful stiffness and infection due to loosening of the attachment between device and bone, instability when the artificial joint dislocates, and wear and tear on plastic parts.

Some other complications are due to double incision surgery and minimally invasive surgery, which seeks to reduce soft tissue damage through reducing the size of the incision. However, implant positioning accuracy and visualization of the bone structures is significantly impaired by these methods. This can result in unintended fractures and soft tissue injury. Surgeons using these approaches are advised to use intraoperative x-ray fluoroscopy or computer guidance systems to guide the surgeon to provide enhanced accuracy. Improved patient outcomes and reduced complications have not been demonstrated when these systems are used when compared to standard techniques.

Further, most metal implant devices have been found to wear down at an accelerated rate in some patients, potentially causing damage and deterioration in the bone and tissue around the implant. There are also concerns that they could leak traces of metal into the bloodstream. In addition, metal surfaces can corrode and release metal ions causing to develop metallosis or metal poisoning.

Many long-term problems with implant devices are the result of osteolysis. This is the loss of bone caused by the body's reaction to polyethylene wear debris, fine bits of plastic that come off the liner over time. An inflammatory process causes bone resorption that may lead to subsequent loosening of the hip implants and even fractures in the bone around the implants. This can also happen with the use of metal and ceramic implants.

In general, post-operative care after implant surgery typically includes periodic doctor visits. Much of this approach relies on the patient to provide feedback to the physician, surgeon, or caretaker should anything out of the ordinary arise. A common problem with an implanted device is that the patient may be unaware of serious problems that are occurring. By the time the patient identifies the problem it may have already escalated to a significant health risk potentially leading to catastrophic consequences. Therefore, it would be desirable to create an *in vivo* and *in vitro* monitoring system and device to monitor physiological and/or mechanical parameters associated with implant devices.

### SUMMARY OF THE INVENTION

The present invention provides an implant device, said implant device comprising one or more structural components being designed to assist in carrying out a biological function of a subject; and one or more capacitive proximity sensors being designed to be powered wirelessly and to detect at least one signal indicative of one or more parameters as a result of a change in capacitance in response to a change in the mechanical characteristics of the one or more components and/or the physiological characteristics of the subject and to produce an output indicative thereof.

In another embodiment, the present invention provides an implant device according to the preceding embodiment, wherein the one or more wirelessly powered capacitive proximity sensors being designed to communicate a data set representative of the output wirelessly to a remote element located outside of the subject.

In further embodiment, the present invention provides an implant device according to any of the preceding embodiments, wherein said implant device comprising at least two components.

In another embodiment, the present invention provides an implant device according to any of the preceding embodiments, wherein the at least two components comprising the one or more capacitive proximity sensors.

In another embodiment, the present invention provides an implant device according to any of the preceding embodiments, wherein the implant device comprising two or more of the capacitive proximity sensors and said sensors being designed to further communicate with each other and to produce an output indicative thereof. Optionally, at least two sensors are paired up.

In another embodiment, the present invention provides an implant device according to any of the preceding embodiments, wherein the one or more wirelessly powered capacitive proximity sensors comprising memory storage to store the data set representative of the output of the one or more wirelessly powered capacitive proximity sensors.

In another embodiment, the present invention provides an implant device according to any of the preceding embodiments, wherein the one or more wirelessly powered capacitive proximity sensors comprising a coating consistent with a coating elsewhere in the implant device.

In further embodiment, the present invention provides an implant device according to any of the preceding embodiments, wherein the components are monoblock and/or modular.

In further embodiment, the present invention provides an implant device according to any of the preceding embodiments, wherein the one or more capacitive proximity sensor comprising a wirelessly powered Radio Frequency Identification circuit.

The present invention further provides, a system providing *in vivo* or *in vitro* diagnostics of one or more parameters. Said system comprising (a) an implant device comprising one or more structural components being designed to assist in carrying out a biological function of a subject; and one or more capacitive proximity sensors being designed to be powered wirelessly and to detect at least one signal indicative of one or more parameters as a result of a change in capacitance in response to a change in the mechanical characteristics of the one or more components and/or the physiological characteristics of the subject and to produce an output indicative thereof; and (b) at least one remote element being designed to transmit an electrical field in the space between the structural components of the implant device and/or in the space between the subject and the structural component.

In further embodiment, the present invention provides a system according to the preceding embodiment, wherein the remote element wirelessly powers the capacitive proximity sensor.

In further embodiment, the present invention provides a system according to the any of the preceding embodiments, the subject comprising one or more of the capacitive proximity sensors.

In further embodiment, the present invention provides a system according to the any of the preceding embodiments, wherein the capacitive proximity sensors being designed to communicate with each other and to produce an output indicative thereof. Additional embodiments provide methods of *in vivo* and *in vitro* diagnostics of the implant device and one or more of said parameters.

### DRAWINGS

Those of skill in the art will understand that the drawings, described below, are for illustrative purposes only. The drawings are not intended to limit the scope of the present teachings in any way.
FIG. 1 depicts a hip implant with integrated capacitive proximity sensors (5), (6), (7), and (8) as part of its structure.
FIG. 2 depicts a cross-sectional view of the hip implant with integrated capacitive proximity sensors (5), (6), (7), and (8) as part of its structure.

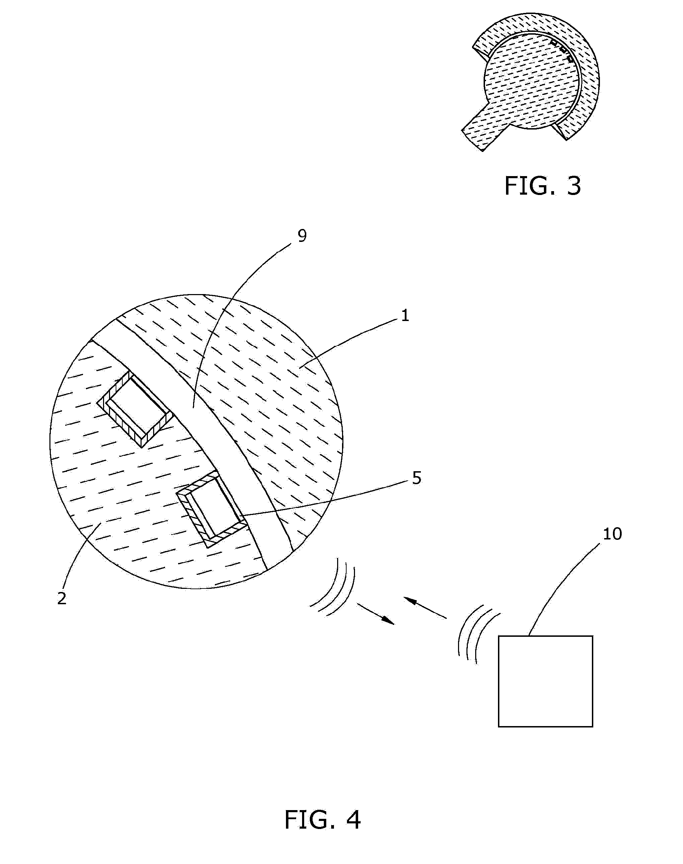

FIG. 3 depicts acetabular shell and femoral head components of the hip implant wherein the femoral head component comprises the capacity proximity sensors as part of its structure.
FIG. 4 illustrates a close-up view of the acetabular shell (1) and the femoral head (2) components of a hip implant separated by gap (9). The femoral head (2) comprises capacitive proximity sensors as part of its structure, for example a sensor (5), which is in communication with the remote element (10).
FIG. 5 depicts acetabular shell and femoral head components of the hip implant wherein both components comprise the capacity proximity sensors as part of their structures.
FIG. 6 illustrates a close-up view of the acetabular shell (1) and the femoral head (2) components separated by gap (9). The acetabular shell (1) comprises capacitive proximity sensors, for example sensor (6) and the femoral head (2) comprises capacitive proximity sensors, for example sensor (5), which the sensors are in communication with the remote element (10).
FIG. 7 depicts the femoral stem (3) of a hip implant and the bone (4) of a subject comprising capacitive proximity sensors.
FIG. 8 illustrates a close-up view of the femoral stem (3) of a hip implant and the bone (2) of a subject separated by gap (9). The femoral stem (3) comprises capacitive proximity sensor (7) and the bone (4) comprises capacitive proximity sensor (8), which the sensors are in communication with the remote element (10).
FIG. 9 depicts a close-up view of the acetabular shell (1) and the femoral head (2) components of the implant separated by gap (9) comprising capacitive proximity sensors (6) and (5), respectively. The capacitive proximity sensors (6) and (5) comprise a conductor plate (12), dielectric (11) and (15), memory storage or chip (14), resistors and coils and antenna (13).
FIG. 10 depicts a close-up view of acetabular shell (1) and the femoral head (2) components of the implant separated by gap (9) and a capacitive proximity sensor integrated within the structure of the femoral head (2) of the implant. The sensor comprises conductor plates (12), (22), dielectrics (18), (19) and (20), memory storage (23), resistors and coils and antenna (21).
FIG. 11 depicts circuit schematic of the capacitive proximity sensor as illustrated in Fig. 10.

### DETAILED DESCRIPTION OF THE INVENTION

In one aspect of the invention, an implant device is provided. The implant device comprising one or more structural components being designed to assist in carrying out a biological function of a subject; and one or more capacitive proximity sensors being designed to be powered wirelessly and to detect at least one signal indicative of one or more parameters as a result of a change in capacitance in response to a change in the mechanical characteristics of the one or more components and/or the physiological characteristics of the subject and to produce an output indicative thereof.

As used herein the term "subject" refers to any animal, such as a mammal like livestock, pets, and preferably a human. Specific examples of "subject" include, but are not limited, to individuals requiring an implant device, and more particularly requiring artificial joint. The term does not denote a particular age or sex. The subject also refers to bone, tissue, and/or organ of any animal, preferably of a human.

As used herein the term "biological function" refers to the structural or mechanical activity of a bone, tissue, and/or organ.

The term "parameter" or "parameters" as used herein, refers to physiological and/or mechanical characteristics of the subject and the implant device, respectively. The physiological characteristics of the subject are indicative of, for example, infection, inflammation, bone corrosion, blood poisoning, and/or osteloysis. The mechanical characteristics of the implant are indicative of, for example, wear of the component(s) of the implant device, fatigue of the component(s) the implant device, corrosion of the component(s) of the implant device, misalignment of the component(s) of the implant device, location and distance of each component relative to the other component(s), the and/or improper implantation of the component(s) of the implant device.

The implant device of the invention can be a monoblock component or modular implant device. Monoblock, One-component or single-piece implant refers to an implant that is designed in one-piece. For example, in hip implant, the acetabular components, the liner, cup, and ball are designed as one piece without using screws, a linker, or locking mechanism to hold them in place. Modular or Two or more component or multiple component implant refers to an implant comprising a number of interchangeable and detachable components. For example, the components of a hip implant, mainly, the acetabular shell, the acetabular liner, the femoral head and the femoral hip stem that are detachable and hold in place by a locking mechanism. Optionally, the component is an artificial joint and more particularly a shoulder joint, an elbow joint, a wrist joint, a hip joint, a knee joint, a finger joint and/or an ankle joint.

As used herein the term "capacitive proximity sensor" refers to a technology that is designed to detect at least one signal indicative of one or more parameters as a result of a change in capacitance in response to a change in the mechanical characteristics of the one or more components of the implant device and/or the physiological characteristics of the subject and to produce an output indicative thereof.

The sensor of the present invention is designed to sense, detect, read, monitor, or simply is responsive to one or more parameters and to transmit or produce a measurable output.

Optionally, the sensor of the present invention is designed to be any electromagnetic wave-emitting, electromagnetic wave-detecting, and/or electromagnetic wave-activated device, such as, for example, radio frequency identification (RFID) transponders or tags, microwave tags, inlays, etc. Optionally, the sensor of the present invention being designed to comprise a wirelessly powered Radio Frequency Identification circuit. Optionally, the sensor of the present invention being designed to further comprise of memory storage or chip to store the sensor's output or output of other sensor and remote element. Optionally, the sensor of the present invention is being designed to be wirelessly powered through external sources, for example, radio frequency power, inductive power, and/or optical power. Optionally, the sensor of the present invention is coupled to other proximity sensor such as inductor sensor and designed to produce an output thereof. Optionally, the sensor of the present invention comprises a communication element, comprising antenna, coil, and resistors designed to communication with a remote element and other sensors and to produce an output thereof. Optionally, the two or more sensors of the present invention are paired up. The term "paired up" or "lined up" and similar terms shall mean that the sensors are in direct communication with each other, which can be used, for example, to monitor and detect position and distance of one sensor with respect to the other and to produce an output. By pairing up the sensors, for example, misalignment and improper implantation can be detected and can assist in initial implant placement.

The number and location of capacitive proximity sensors within the implant or its component(s) may vary. Placing the sensor on the subject, like the bone is optional. The term "on" in relation with placement of the sensors includes "in", "within", and/or part of the structure of the component as long as they are designed and operatively capable of detecting and transmitting one or more parameters.

The present invention further provides a system for *in vivo* or *in vitro* diagnostics of one or more parameters. Said system comprising (a) an implant device comprising one or more structural components being designed to assist in carrying out a biological function of a subject; (b) one or more capacitive proximity sensors being designed to be powered wirelessly and to detect at least one signal indicative of one or more parameters as a result of a change in capacitance in response to a change in the mechanical characteristics of the one or more components and/or the physiological characteristics of the subject and to produce an output indicative thereof; and (c) at least one remote element being designed to transmit an electrical field in the space between the components of the implant device and/or in the space between the subject and the structural component.

The implant device, the sensor, and the parameters of the system of present invention are in accordance with any of the preceding embodiments.

The remote element of the present invention is optionally located outside the subject. The remote element of the present invention is being designed to sense, detect, read, monitor, or simply is responsive to one or more of the sensors of the present invention and to produce a measurable output. Optionally, the remote element is being designed to wirelessly power the sensors. Optionally, the remote element of the present invention is designed to be any electrical filed emitting, electromagnetic wave-emitting, electromagnetic wave-detecting, and/or electromagnetic wave-activated and - activating device, such as, for example, radio frequency identification (RFID) transponders or tags, microwave tags, inlays, etc. More than one remote element can be used to transmit the signal to different sensors placed at different locations.

The output of the sensor and/or the remote element of the present invention may be measured and displayed by various types of consumer electronics devices, including, but not limited to, personal computers, digital video disk devices, television sets, audio reproduction systems, video tape recorders (VCRs), smart phones, and notebooks. However, in various alternate embodiments, any appropriate electronic network designed to permit communication between any desired types of electronic devices. The present invention further provides a method for *in vivo* or *in vitro* diagnostics of one or more parameters. Said method comprising transmitting an electrical field in the space between the components of the implant device according to any of the preceding embodiments and/or in the space between the subject and the component of the implant device according to any of the preceding embodiments; wherein the implant device comprises one or more structural components being designed to assist in carrying out a biological function of a subject and one or more capacitive proximity sensors being designed to be powered wirelessly and to detect at least one signal indicative of one or more parameters as a result of a change in capacitance in response to a change in the mechanical characteristics of the one or more components and/or the physiological characteristics of the subject and to produce an output indicative thereof; and analyzing the output of the sensors generated by transmitting the electrical filed to determine the one or more parameters. Optionally, analyzing involves comparing the output values with predetermined values. For example, upon placement of the implant in the subject, the remote element transmits a signal, for example, an electrical filed to the implant device, whereby a predetermined set of values, or initial values based on the measured capacitance are obtained. Subsequent transmission of the signal or an electrical field generates subsequent values, which will be compared with the predetermined values to determine the one or more parameters.

### Example:

### Detecting and monitoring parameters of hip joint implant

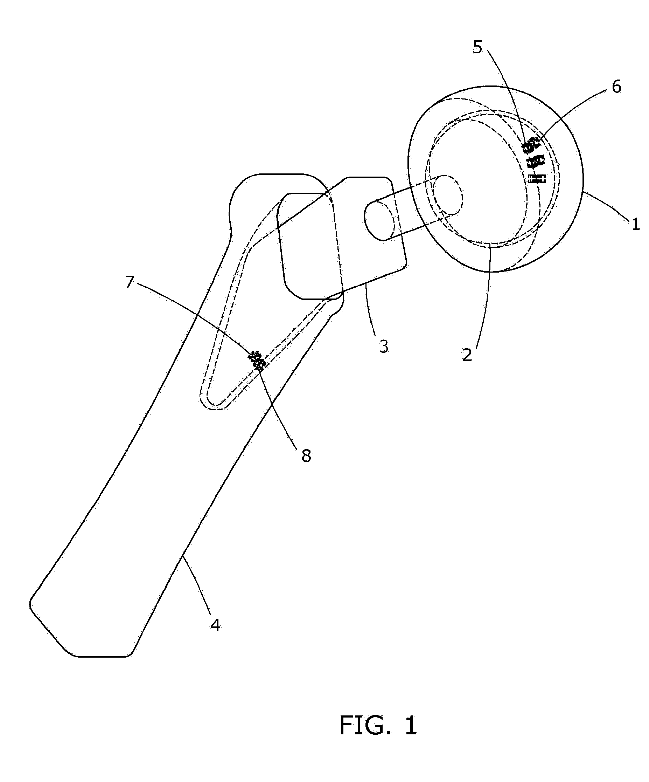

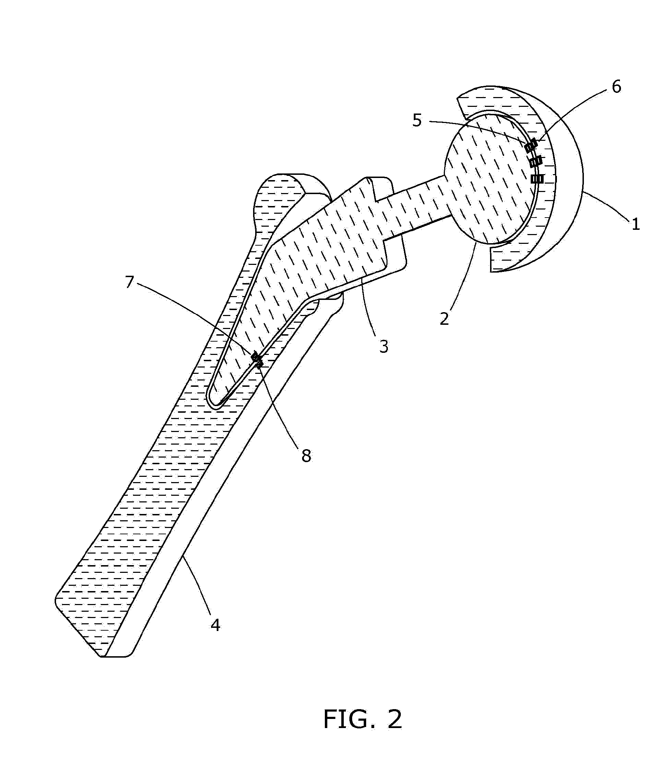

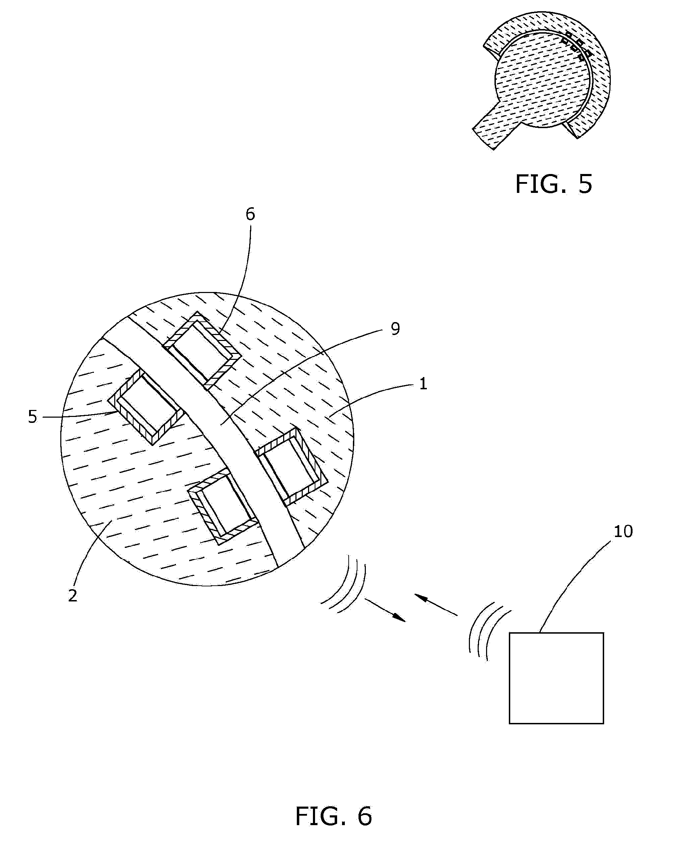

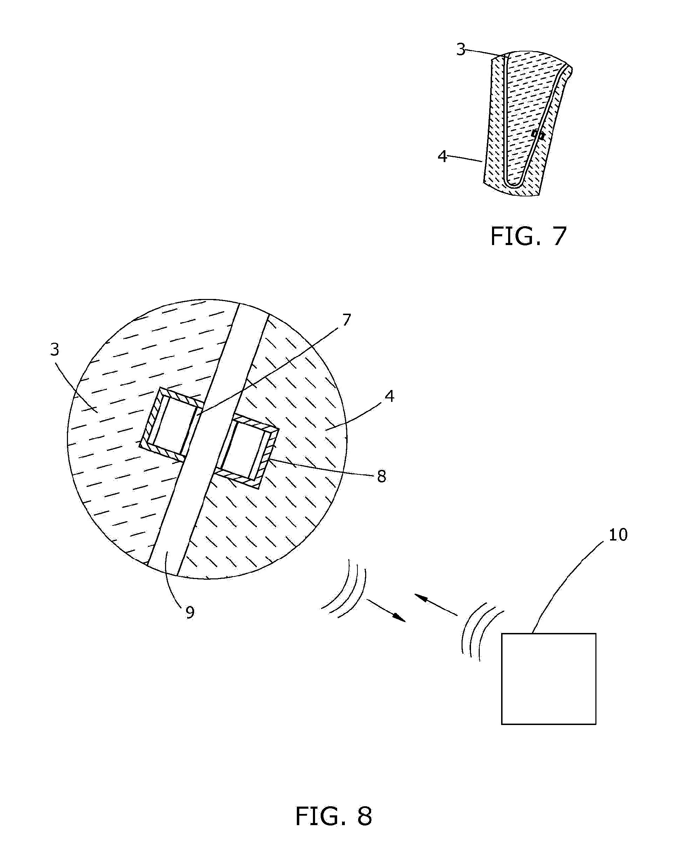

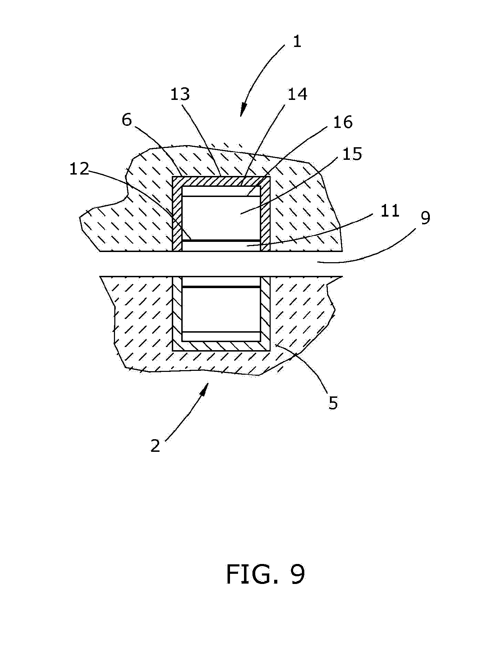

As illustrated by Figure 1 and 2, the modular components of a hip implant include acetabular shell (1), acetabular liner (not show), femoral head (2) and femoral hip stem (3). The implant as part of its structure contains three capacitive proximity sensors (6) on the acetabular shell (1) and three capacitive proximity sensors (5) on the femoral head (2), one capacitive proximity sensor (7) on the femoral stem and a capacitive proximity sensor (8) on the bone. As illustrated in Fig. 6 and Fig. 8, the remote element (10) transmits a radio frequency signal via RFID to the mentioned sensors; thereby the sensors generate an output. The output is result of a possible change in capacitance in response to a change in the mechanical characteristics of the shell (1), the head (2), the stem (3) and/or the physiological characteristics of the bone (4) and to produce an output indicative thereof. In this example the capacitive proximity sensors have one conducting element 12 that detects the change in capacitance. For example, the conducting element 12 of the sensor 6, measures the capacitance between the dielectric (11) and the surface of the shell (1). If there is a wear or inflammation on the surface of the shell (1), the dielectric (11) will change its conformation causing the capacity to change. In another example, the conducting element (12) of the sensor (6) is placed on the surface of the implant designed to detect the change in capacitance as result of change in conformation of the dielectric material or gap (9). Optionally, the gap 9 includes a polyethylene insert or liner. For example, leakage of materials, i.e. debris or parts of the materials from component (1) or (2) within the gap (9) or from the insert, causes change in the capacitance. The sensor (6) transmits a measurable output indicating the change in capacitance to the remote element. In addition, as depicted by figures 2 and 9, the sensors are paired or lined up with each other. In this example, the sensors 5 and 6 are paired up to communicate and to produce a data set representative of the position and distance of each other, in addition to producing a data set representative of other parameters.

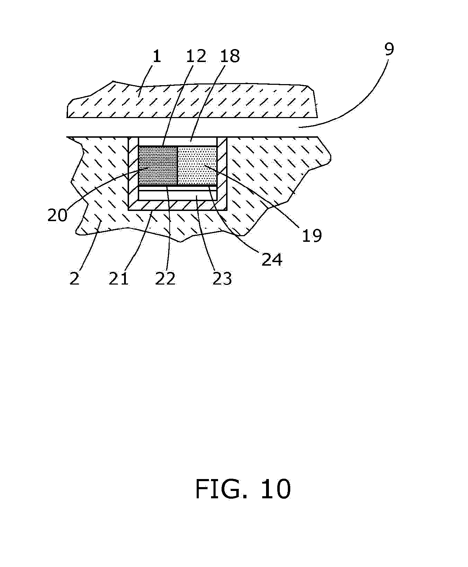

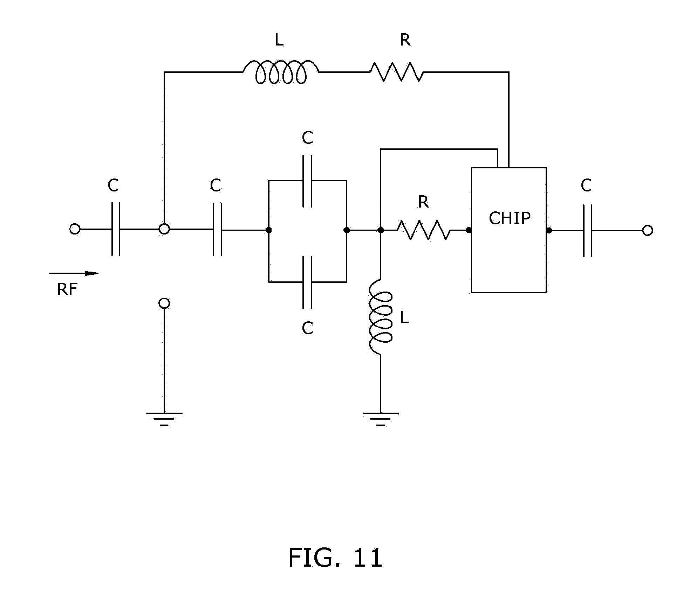

Different capacitive proximity sensors with one or multiple conducting plates, dielectric materials can be placed within the implant as long as they are designed to detect and generate an output indicating the one or more parameters as a result of a change in capacitance in response to a change in the mechanical characteristics of the one or more components and/or the physiological characteristics of the subject and to produce an output indicative thereof. For example, Fig. 10 depicts a capacitance proximity sensors with multiple conducting plates such as (12) and (22) that can detect the capacitance and/or change in capacitance caused by change in conformation of dielectric materials (19), (20), (18), or (9) or combinations thereto when an electrical current is transmitted to the sensor. In this example, the sensor comprises a chip (23) to store the data representative the output of the sensor and to transmit the data to the remote element via the communication element (21) containing antenna, coil and resistors. Fig. 11 depicts the circuit schematic of the capacitive proximity sensor as illustrated in Fig. 10 when exposed to the Radio Frequency (RF) signal, which produces the measurable output.

### OTHER EMBODIMENTS

The detailed description set-forth above is provided to aid those skilled in the art in practicing the present invention. However, the invention described and claimed herein is not to be limited in scope by the specific embodiments herein disclosed because these embodiments are intended as illustration of several aspects of the invention. The embodiments set-forth above can be performed and combined with other disclosed embodiments according to the invention. Any equivalent embodiments are intended to be within the scope of this invention. Indeed, various modifications of the invention in addition to those shown and described herein will become apparent to those skilled in the art from the foregoing description which do not depart from the spirit or scope of the present inventive discovery. Such modifications are also intended to fall within the scope of the appended claims.

The phrase "and/or" as used herein in the specification and in the claims, should be understood to mean "either or both". As a non-limiting example, a reference to " the monoblock and/or modular" can refer, in one embodiment, to the monoblock component only (optionally including elements other than modular); in another embodiment, to modular component only (optionally including elements other than the monoblock component); in yet another embodiment, to both the monoblock and modular components (optionally including other elements); etc.

## Claims

1. An implant device, comprising:
a. One or more structural components being designed to assist in carrying out a biological function of a subject; and
b. One or more capacitive proximity sensors being designed to be powered wirelessly and to detect at least one signal indicative of one or more parameters as a result of a change in capacitance in response to a change in the mechanical characteristics of the one or more components and/or the physiological characteristics of the subject and to produce an output indicative thereof.

2. The implant device of claim 1, wherein the one or more wirelessly powered capacitive proximity sensors being designed to communicate a data set representative of the output wirelessly to a remote element located outside of the subject.

3. The implant device of claim 1, wherein the implant device comprising at least two structural components, wherein the at least two structural components comprising the one or more capacitive proximity sensors, and wherein at least two of said sensors are paired up.

4. The implant device of claim 1, wherein the one or more wirelessly powered capacitive proximity sensors comprising a memory storage being designed to store a data set representative of the output of the one or more wirelessly powered capacitive proximity sensors.

5. The implant device of claim 1, wherein the one or more wirelessly powered capacitive proximity sensors comprising a coating consistent with a coating elsewhere in the implant device.

6. The implant device of claim 1, wherein the one or more capacitive proximity sensor comprising a wirelessly powered Radio Frequency Identification circuit.

7. A system for providing *in vivo* or *in vitro* diagnostics of one or more parameters, comprising:
a. An implant device comprising one or more structural components being designed to assist in carrying out a biological function of a subject;
b. One or more capacitive proximity sensors as part of structure of the implant device being designed to be powered wirelessly and to detect at least one signal indicative of one or more parameters as a result of a change in capacitance in response to a change in the mechanical characteristics of the one or more components and/or the physiological characteristics of the subject and to produce an output indicative thereof; and
c. At least one remote element being designed to transmit an electrical field in the space between the components of the implant device and/or in the space between the subject and the component.

8. The system of claim 7, wherein the remote element wirelessly powers the capacitive proximity sensor.

9. The system of claim 7, wherein the subject comprising one or more of the capacitive proximity sensors.

10. The implant device of claim 1 and the system of claim 7, wherein the parameter is indicative of wear of the implant device, fatigue of the implant device, corrosion of the implant device, misalignment of the implant device, infection, osteolysis or combinations thereof.

11. The implant device of claim 1 and the system of claim 7, wherein the component comprises an artificial joint.

12. The implant device and system of claim 11, wherein the joint is selected from a group consisting of a shoulder joint, an elbow joint, a wrist joint, a hip joint, a knee joint, and an ankle joint.
